(19) Europäisches Patentamt
European Patent Office
Office européen des brevets

(11) Veröffentlichungsnummer: **0 036 169**
**B1**

(12) # EUROPÄISCHE PATENTSCHRIFT

(45) Veröffentlichungstag der Patentschrift: **29.06.83**

(51) Int. Cl.³: **C 07 D 311/58**, C 07 D 311/20, C 07 D 311/66, C 08 K 5/15

(21) Anmeldenummer: **81101735.9**

(22) Anmeldetag: **10.03.81**

(54) **Chromanderivate, Verfahren zu ihrer Herstellung, ihre Verwendung als Stabilisatoren von organischen Materialien sowie diese Stabilisatoren enthaltende organische Materialien.**

(30) Priorität: **19.03.80 DE 3010505**

(43) Veröffentlichungstag der Anmeldung: **23.09.81 Patentblatt 81/38**

(45) Bekanntmachung des Hinweises auf die Patenterteilung: **29.06.83 Patentblatt 83/26**

(84) Benannte Vertragsstaaten: **BE CH DE FR GB IT LI NL**

(56) Entgegenhaltungen:
**AT-B-210 625**
**DD-A-109 624**

(73) Patentinhaber: **BASF Aktiengesellschaft, Carl-Bosch-Strasse 38, D-6700 Ludwigshafen (DE)**

(72) Erfinder: **Nissen, Axel, Dr., Panoramastrasse 51, D-6906 Leimen (DE)**
Erfinder: **Horner, Michael, Dr., Im Ritterbueschel 9, D-6730 Neustadt (DE)**
Erfinder: **Horn, Dieter, Dr., Schroederstrasse 69, D-6900 Heidelberg 1 (DE)**
Erfinder: **Lueddecke, Erik, Dr., Altholzweg 25, D-6700 Ludwigshafen (DE)**
Erfinder: **Teege, Gernot, Dr., Alwin-Mittasch-Platz 12, D-6700 Ludwigshafen (DE)**

**EP 0 036 169 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Chromanderivate, Verfahren zu ihrer Herstellung, ihre Verwendung als Stabilisatoren von organischen Materialien sowie diese Stabilisatoren enthaltende organische Materialien

Die vorliegende Erfindung betrifft neue Chromanderivate der allgemeinen Formel I

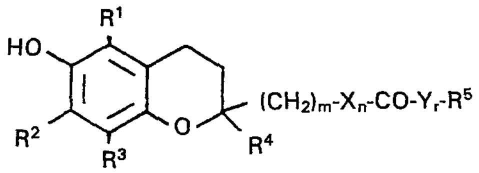

I

In dieser Formel bedeuten:

$R^1$-$R^4$ H, $C_1$-$C_4$-Alkyl
$R^5$ $C_{10}$-$C_{30}$-Alkyl oder -Alkenyl
X, Y O, NH, S
m 0, 1, 2 oder 3
n, r 0 oder 1

Ausserdem betrifft die Erfindung die Herstellung der Verbindungen I, deren Verwendung als Stabilisatoren von organischen Massen, vor allem von Kunststoffen, gegen die Einflüsse von Licht, Wärme und Oxidationsmitteln, sowie die mit diesen Stabilisatoren stabilisierten organischen Massen.

Aus der DE-A-2 364 165 sind Chromanderivate des Typs I′ bekannt,

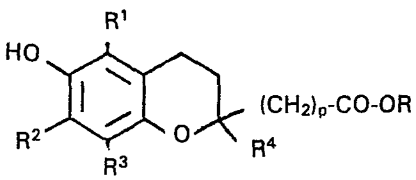

I′

in denen p = 0 oder 1 ist und R eine $C_1$-$C_7$-Alkylgruppe bedeutet. Die erfindungsgemässen Chromanderivate I unterscheiden sich von den bekannten Chromanderivaten I′ im wesentlichen durch die $C_{10}$-$C_{30}$-Alkylgruppe anstelle der $C_1$-$C_7$-Alkylgruppe R. Ähnliche Verbindungen I″

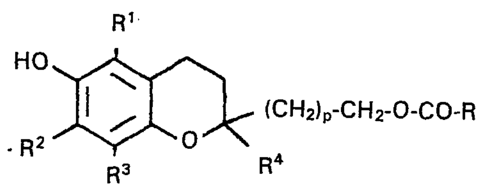

I″

also solche, die ebenfalls einen kurzkettigen Rest R enthalten, sind aus der DE-A-2 364 141 bekannt.

Die Verbindungen I′ und I″ lassen in ihren Eigenschaften zur Stabilisierung von Kunststoffen und sonstigen organischen Massen indes zu wünschen übrig. Insbesondere werden die guten Stabilitätswerte, die mit dem strukturverwandten α-Tocopherol (I‴, Vitamin E)

I‴

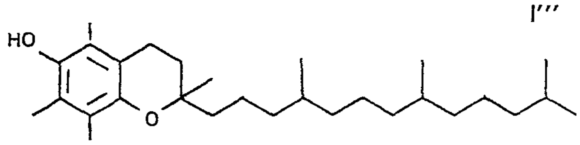

erzielt werden, wie z.B. aus den DE-C-1 114 319 und 1 136 102 bekannt ist, nicht erreicht.

α-Tocopherol ist jedoch nur relativ schwierig herzustellen und deshalb als Stabilisierungsmittel für die meisten Zwecke zu teuer. Ausserdem gibt α-Tocopherol häufig zu unerwünschten Verfärbungen Anlass. Auch in verarbeitungstechnischer Hinsicht bietet die sehr oxidationsempfindliche ölige Substanz α-Tocopherol Probleme.

Der Erfindung lag daher die Aufgabe zugrunde, das α-Tocopherol durch im wesentlichen gleich wirkende, aber billigere Stabilisierungsmittel mit gleichartigen oder besseren Qualitätswerten zu ersetzen.

Es wurde gefunden, dass sich die eingangs definierten neuen Chromanderivate I hervorragend als Stabilisierungsmittel für organische Massen, darunter vor allem für Kunststoffe, eignen.

Ferner wurden verschiedene, im folgenden näher beschriebene Verfahren für die Herstellung der Chromanderivate I gefunden. Unter den Verbindungen I werden solche bevorzugt, in denen die Reste $R^1$-$R^4$ Methylgruppen bedeuten, da Chromanderivate dieser Struktur besonders leicht zugänglich sind. Der Chromanrest mit dieser Struktur

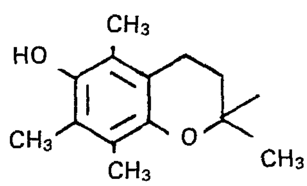

A

wird daher im folgenden als Rest A definiert, so dass die Verbindungen I auch kurz als

$$A\text{-}(CH_2)_m\text{-}X_n\text{-}CO\text{-}Y_r\text{-}R^5 \qquad I$$

bezeichnet werden können. Sofern $R^1$-$R^4$ andere Reste als die Methylgruppe bezeichnen, wird dies durch einen Vermerk wie «A ($R^4$ = H)» kenntlich gemacht, was in diesem Falle bedeutet, dass die Reste $R^1$-$R^3$ für Methylgruppen und der Rest $R^4$ für Wasserstoff stehen.

Verbindungen I mit anderen Resten $R^1$-$R^4$ als jeweils der Methylgruppe sind in analoger Weise zugänglich, wie diejenigen mit der Gruppierung A, und ihre Wirkung als Stabilisierungsmittel ist nach den bisherigen Beobachtungen etwa die gleiche wie bei den Tetramethylchromanderivaten.

Wesentlich für die Stabilisierungswirkung ist der Alkyl- bzw. Alkenylrest $R^5$, der definitionsgemäss 10 bis 30 C-Atome enthalten soll, wobei lineare Reste und methylverzweigte Reste bevorzugt werden. Die Art der Gruppierung $-X_n\text{-}CO\text{-}Y_r-$ ist im Hinblick auf die Stabilisierungseigenschaften von I von untergeordneter Bedeutung, denn diese Gruppierung hat in erster Linie die Funktion, den Rest $R^5$ mit der Chromangruppierung zu verknüpfen. Auch von der Anzahl m der Methylgruppen werden die Stabilisierungseigenschaften nicht erkennbar beeinflusst. Die sonstigen Eigenschaften, u.a. die Verträglichkeit und Mischbarkeit mit dem Substrat, können jedoch

von m sowie von der Gruppierung $-X_n-CO-Y_r-$ abhängen. Beispielsweise werden für die Stabilisierung von Polyamiden Verbindungen I, in denen X und/oder Y für eine -NH-Gruppierung steht, aus Gründen der Verarbeitungstechnik bevorzugt, und für hydrophobe Kunststoffe wie Polypropylen empfehlen sich Verbindungen I mit Esterstruktur (X oder Y = O).

Der Art der Gruppierung $-X_n-CO-Y_r-$ entsprechen die folgenden 16 Verbindungstypen Ia-Ip:

| | | |
|---|---|---|
| Ia | n = r = 0 | -CO- |
| Ib | n = 0, Y = O | -CO-O- |
| Ic | n = 0, Y = NH | -CO-NH- |
| Id | n = 0, Y = S | -CO-S- |
| Ie | X = O, r = 0 | -O-CO- |
| If | X = O, Y = O | -O-CO-O- |
| Ig | X = O, Y = NH | -O-CO-NH- |
| Ih | X = O, Y = S | -O-CO-S- |
| Ii | X = NH, r = 0 | -NH-CO- |
| Ij | X = NH, Y = O | -NH-CO-O- |
| Ik | X = NH, Y = NH | -NH-CO-NH- |
| Il | X = NH, Y = S | -NH-CO-S- |
| Im | X = S, r = 0 | -S-CO- |
| In | X = S, Y = O | -S-CO-O- |
| Io | X = S, Y = NH | -S-CO-NH- |
| Ip | X = S, Y = S | -S-CO-S- |

Für die Herstellung dieser Verbindungen gilt allgemein das Prinzip, Chromanderivate des Typs

$$A-(CH_2)_m-CO-Y-H \quad \text{oder} \quad A-(CH_2)_m-XH$$

mit Verbindungen $R^5$-OH bzw. $R^5-Y_2-CO-OH$ oder deren funktionellen Derivaten wie Chloriden oder Methylestern umzusetzen.

Ferner kann man auch von Chroman-Vorprodukten des Typs

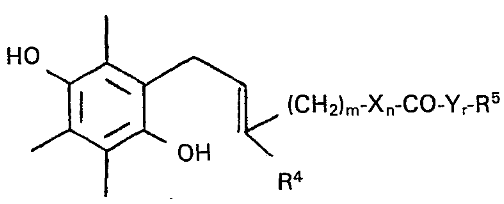

ausgehen und den Ringschluss über das hieraus herzustellende Chinon anschliessend vornehmen und das entstehende Chrom-2-en hydrieren. Die Verknüpfung des Chromanteils des Moleküls mit dem Rest $R^5$ über die $-X_n-CO-Y_r$-Gruppierung wird dabei in analoger Weise vorgenommen, wie sie vorstehend in allgemeiner Form beschrieben wurde.

Bei allen diesen Synthesen kann es sich ferner empfehlen, die 6-OH-Gruppe in bekannter Weise durch einen leicht wieder abspaltbaren Rest, z.B. die Benzylgruppe, zu schützen und diesen Rest im letzten Syntheseschritt zu eliminieren.

Im einzelnen sind die Verbindungen Ia-Ip nach folgenden Methoden (a)-(p) erhältlich:

(a) Herstellung der Ketone Ia

Man geht von einem Säurehalogenid IIa aus

$$A-(CH_2)_m-CO-Hal \qquad IIa$$

in welchem die 6-OH-Gruppe durch eine Schutzgruppe, vorzugsweise die Benzylgruppe, geschützt ist, und setzt dieses in bekannter Weise in Gegenwart von $CdCl_2$ mit einer Grignard-Verbindung $R^5$-MgHal um. Das Addukt wird danach unter Abspaltung der Schutzgruppe hydrolysiert. Die Aufarbeitung auf Ia erfolgt dann wie üblich.

Es ist auch möglich, die Schutzgruppe nach der Hydrolyse hydrogenolytisch abzuspalten.

Die Säurehalogenide IIa sind aus der DE-A-2 364 141 bekannt oder hiernach erhältlich.

(b) Herstellung der Ester Ib

($b_1$) Veresterungsverfahren

Die Ester Ib sind besonders leicht durch übliche, sauer katalysierte Veresterung der Carbonsäuren IIb

$$A-(CH_2)_m-COOH \qquad IIb$$

mit Alkoholen $R^5$-OH (IIIb) erhältlich. Die Ausgangsverbindungen IIb sind z.B. aus der DE-A-2 364 141 bekannt.

Die Veresterung ist in ihren zahlreichen Ausgestaltungen hinsichtlich der Säure, der Entfernung des Reaktionswassers, der Temperatur und der Reaktionszeit allgemein bekannt, so dass nähere Ausführungen sich hierzu erübrigen. Es ist lediglich hinzuzufügen, dass es sich meistens empfiehlt, die Säure IIb im Überschuss einzusetzen. Die nicht verbrauchte Säure kann dann mit wässrigem Alkali vom Ester Ib abgetrennt werden, wonach die Säure IIb durch Ansäuern der wässrig-alkalischen Lösung wieder leicht zurückgewonnen werden kann. Die Reinigung von Ib kann, falls überhaupt erforderlich, durch Umkristallisation vorgenommen werden, wozu sich meistens Methanol/Wasser-Gemische eignen. Anstelle der Alkohole $R^5$-OH kann man auch die Halogenide $R^5$-Hal oder die Ester von anorganischen oder organischen Säuren einsetzen. In diesem Falle nimmt man die Veresterung bzw. Umesterung in Gegenwart von schwachen Basen sowie zweckmässigerweise in einem Lösungsmittel vor. Auch diese Methode ist allgemein bekannt, so dass sich nähere Ausführungen hierzu erübrigen. Umgekehrt kann man auch von den Halogeniden oder Estern von IIb ausgehen und diese mit den Alkoholen IIIb zu den Estern Ib umsetzen.

Eine weitere Möglichkeit zur Herstellung der Ester Ib besteht in der Umsetzung der leicht zugänglichen Nitrile IIb'

$$A-(CH_2)_m-CN \qquad IIb'$$

mit Alkoholen in Gegenwart von Mineralsäuren. Die Herstellung der Nitrile IIb' ist in der älteren Patentanmeldung DE-A1-2 909 601 beschrieben.

($b_2$) Cyclisierungsverfahren

Im Hinblick auf die vom Trimethylhydrochinon (oder seinen Homologen) ausgehende Gesamtsynthese kann es zweckmässig sein, zunächst ein offenkettiges Trimethylhydrochinonderivat herzustellen, welches die Gruppierung $-(CH_2)_m-CO-O-R^5$ enthält, und die Cyclisierung zum Chroman zum Schluss vorzunehmen. Dieser Syntheseweg lässt sich wie folgt veranschaulichen:

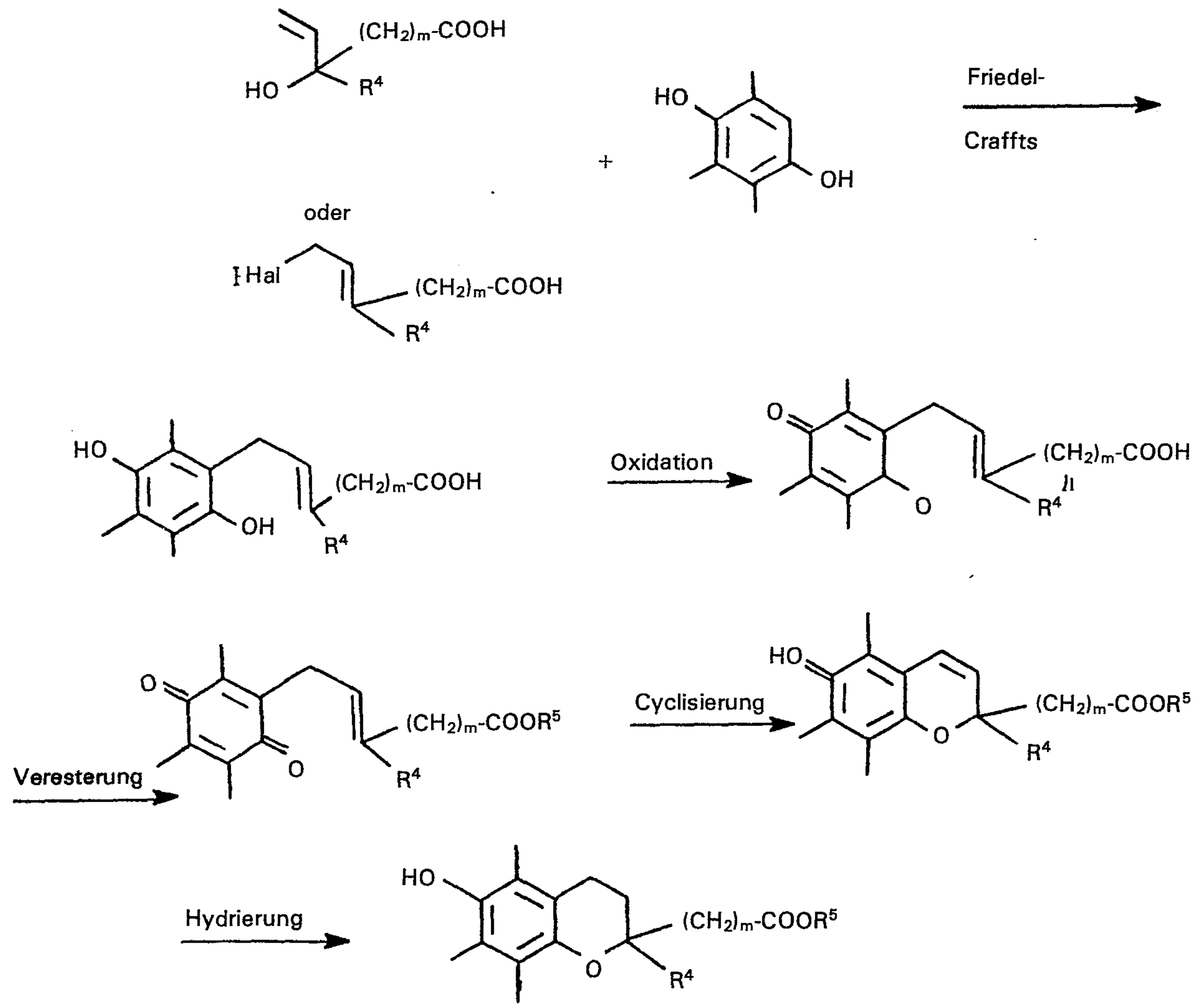


Hierbei kann man den Veresterungsschritt, der nach den unter $(b_1)$ beschriebenen Verfahren vorgenommen werden kann, auch vorverlegen. Die Umsetzung des Trimethylhydrochinons mit den Allylverbindungen erfolgt in bekannter Weise nach den Methoden der Friedel-Craffts-Synthese. Die Oxidation der hierbei entstehenden Zwischenverbindung zum Chinon verläuft leicht und kann z.B. mit Luft vorgenommen werden. Sie ist in der Regel erforderlich, weil nur das Chinon zum Sechsring Chroman cyclisiert werden kann, denn die Cyclisierung des Hydrochinons würde meistens zu einem O-haltigen Fünfring führen. Die Cyclisierung nimmt man ebenfalls wie üblich vor, z.B. mittels einer tertiären Stickstoffbase wie Triethylamin oder Pyridin.

Das gleiche gilt für die anschliessende Hydrierung des hierbei entstehenden Chromanderivates.

(c) Herstellung der Amide Ic

Hierzu setzt man die Carbonsäuren IIb oder deren funktionelle Derivate wie die Halogenide oder Ester nach bekannten Methoden mit einem Amin $R^5$-$NH_2$ um.

(d) Herstellung der Thioester Id

Die Herstellung der Thioester Id verläuft analog der Veresterung Ib, indem man statt von den Alkoholen $R^5$-OH von den Thiolen $R^5$-SH ausgeht.

(e) Herstellung der Ester Ie

Ausgangsverbindungen sind in diesem Falle die Chromanderivate IIe

$A-(CH_2)_m-OH$ IIe

die man beispielsweise durch Friedel-Crafts-Addition von Diolen

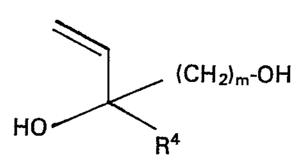

an Trimethylhydrochinon erhält.

Die Veresterung von IIe und Ie erfolgt dann wie üblich mit einer Säure $R^5$-COOH (IIIe) oder einem funktionellen Derivat hiervon.

(f) Herstellung der Carbonate If

Hierzu setzt man die Verbindungen IIe analog der Methode (e) mit einem Chlorameisensäureester $R^5$-O-CO-Hal (IIIf) oder einem Carbonat wie $R^5$-O-CO-$CH_3$ um.

(g) Herstellung der Urethane Ig

Die einfachste Methode zur Herstellung dieser Verbindungen besteht in der Addition eines Isocyanats $R^5$-N=C=O (IIIg) an einen Alkohol IIe, wobei es sich allerdings empfiehlt, die 6-OH-Gruppe des Chromans zu schützen.

Anstelle des Isocyanats IIIg kann man auch ein entsprechendes Carbaminsäurehalogenid $R^5$-NH-CO-Hal oder ein Urethan wie $R^5$-NH-CO-O-$CH_3$ einsetzen.

(h) Herstellung der Monothiocarbonate Ih

Diese Verbindungen erhält man nach der Methode (f), mit dem Unterschied, dass man hier die entsprechenden Thioderivate (IIIh) also $R^5$-S-CO-Hal oder $R^5$-S-CO-O-$CH_3$ einsetzt.

(i) Herstellung der Amide Ii

Ausgangsverbindungen sind die Amine IIi

$$A\text{-}(CH_2)_m\text{-}NH_2 \qquad \text{IIi}$$

die in bekannter Weise durch Hydrolyse der Nitrile IIb' hergestellt werden können.

Die Amine IIi werden sodann in bekannter Weise mittels der Carbonsäure IIIe bzw. deren funktionellen Derivaten in die Amide Ii überführt.

(j) Herstellung der Urethane Ij

Die Urethane Ij sind analog der Methode (f) aus den Aminen IIi und den Chlorameisensäureestern IIIf bzw. deren Carbonaten $R^5$-O-CO-O-$CH_3$ erhältlich.

(k) Herstellung der Harnstoffe Ik

Diese Verbindungen erhält man, indem man die Amine IIi nach den Methoden (g) mit den Isocyanaten IIIg bzw. mit den entsprechenden Carbaminsäurehalogeniden oder Urethanen in bekannter Weise umsetzt.

(l) Herstellung der Thiocarbamate Il

Hierzu setzt man die Amine IIi nach der Methode (h) mit den Thioverbindungen IIIh, $R^5$-S-CO-Hal bzw. $R^5$-S-CO-O-$CH_3$ um.

(m)-(p) Herstellung der Thioverbindungen Im-Ip

Die Herstellung dieser Verbindungen verläuft analog den Methoden (e)-(h), indem man von den entsprechenden Thiolen IIm

$$A\text{-}(CH_2)_m\text{-}SH \qquad \text{IIm}$$

ausgeht, die ihrerseits auf die für die Alkohole IIe genannten Verfahren zugänglich sind.

Die erfindungsgemässen Chromanderivate I eignen sich hervorragend als Stabilisatoren von organischen Materialien gegen Schädigungen durch Wärme, Licht und oxidative Einflüsse. Als organische Materialien sind Fette, Ole, Wachse, pharmazeutische und kosmetische Zubereitungen sowie vor allem Kunststoffe zu nennen. Je nach der Beanspruchung dieser Materialien verwendet man die Stabilisatoren in Konzentrationen von 0,01 bis 1,0, in der Regel von 0,1 bis 0,5 Gew.-%, bezogen auf die Menge des Kunststoffes. Für hochempfindliche Substrate, z.B. Vitamine, können die Konzentrationen bis zu 20 Gew.-% ansteigen. Die erfindungsgemässen Stabilisatoren können allein oder in Mischung mit anderen Stabilisatoren, besonders mit sogenannten Synergisten, verwendet werden. Synergisten sind solche Verbindungen, die für sich allein keine oder nur eine geringe stabilisierende Wirkung haben, zusammen mit Stabilisierungsmitteln deren Effekt jedoch deutlich verbessern. Solche Synergisten sind beispielsweise Calciumstearat und Distearylthiodipropionat

$$S(\text{-}CH_2\text{-}CH_2\text{-}CH_2\text{-}CO\text{-}O\text{-}stearyl)_2$$

Man verwendet die Synergisten im allgemeinen in Mengen von 50 bis 500 Gew.-%, bezogen auf die Menge des Stabilisierungsmittels.

Allgemein werden die organischen Materialien durch Stabilisatoren gegen Veränderungen und Zersetzung geschützt, im Falle von Kunststoffen also vornehmlich gegen den Abbau und die unerwünschte Vernetzung der Makromoleküle, Veränderungen, die sich als Alterung, Versprödung, Verfärbung und Herabsetzung der Erweichungstemperatur äussern.

Für die Eignung und Wirksamkeit von Stabilisatoren sind vor allem folgende Kriterien massgebend:

1. Farbe

Das Substrat soll durch den Stabilisator nicht verfärbt werden. Diese Forderung, die naturgemäss für farblose Kunststoffe von besonderer Wichtigkeit ist, wird durch die erfindungsgemässen Stabilisatoren bei den meisten Kunststoffen befriedigend bis ausgezeichnet erfüllt, und in aller Regel sind diese Verbindungen herkömmlichen Stabilisierungsmitteln, darunter auch dem $\alpha$-Tocopherol, überlegen. Die quantitative Ermittlung der Farbeigenschaften kann nach verschiedenen Methoden bestimmt werden, z.B. nach dem Yellowness-Test (ASTMD 1925).

2. Verarbeitungsstabilität

Hierunter ist die Eigenschaftskonstanz von Thermoplasten gegenüber der mechanischen und thermischen Beanspruchung bei Formgebungsverfahren wie der Extrusion und dem Spritzguss zu verstehen. In dieser Beziehung werden mit den erfindungsgemässen Stabilisatoren besonders gute Ergebnisse erzielt. Eine Masszahl für die Verarbeitungsstabilität lässt sich aus der Änderung des Schmelzverhaltens des betreffenden Thermoplasten nach mehrmaliger Formgebung unter Wiederaufschmelzen ableiten. Der entsprechende Schmelzindex-Text ist in DIN 53 735 beschrieben. Ein wichtiges Kriterium für die Verarbeitungsstabilität ist auch die Farbkonstanz, die z.B. nach dem Yellowness-Test ermittelt werden kann.

3. Langzeitstabilität

Das Verhalten des Kunststoffes gegen rigorose thermische und oxidative Einflüsse ist ein Indiz für die Dauer der Qualitätskonstanz bei bestimmungsgemässem Gebrauch, d.h. aus den bei den entsprechenden Tests (DIN 53 383, Blatt 1) ermittelten Werten lässt sich die Lebensdauer des Kunststoffartikels abschätzen. Hier bieten die erfindungsgemässen Stabilisatoren in Verbindung mit Synergisten Vorteile.

Nähere Angaben zur Qualitätsprüfung der erfindungsgemässen Stabilisatoren sind den Versuchen über deren Wirkung zu entnehmen.

*Beispiele für die Herstellung von Chromanderivaten I*

*Beispiele 1 bis 14*

Herstellung von Chromanderivaten A-$(CH_2)_m$-CO-O-

$-R^5$ (Ib) nach Methode (b)

In den Beispielen 1 bis 12 wurden je 80 g (0,32 Mol) 6-Hydroxy-2,5,7,8-tetramethylchroman-2--carbonsäure

$$A-(CH_2)_n-COOH$$

mit 0,28 Mol eines Alkohols $R^5$-OH so lange in einer Lösung aus 500 ml Toluol und 10 g Phosphorsäure unter Rückflusskühlung erhitzt, bis kein Wasser mehr abgeschieden wurde. Anschliessend wurde die überschüssige Säure mit 400 ml wässriger $NaHCO_3$-Lösung von der organischen Phase abgetrennt, welche wie üblich aufgearbeitet wurde. Der rohe Ester Ib wurde sodann aus Methanol/Wasser umkristallisiert.

In den Beispielen 13 und 14 wurden je 10 g (43 mMol) des Nitrils A-CN in 50 ml wasserfreiem Tetrahydrofuran und 30 g des Alkohols $R^5$-OH gelöst. Diese Lösung wurde bei 0°C mit Chlorwasserstoff gesättigt und 3 Tage bei dieser Temperatur gehalten. Die Aufarbeitung dieses Reaktionsgemisches erfolgte wie üblich.

Die Ergebnisse sind in Tabelle 1 zusammengestellt. Soweit im Rest A einer der Substituenten $R^1$-$R^4$ nicht die Methylgruppe bedeutet, ist dies durch Angabe des tatsächlichen Substituenten, der anstelle der Methylgruppe steht, vermerkt.

Tabelle 1

Verbindungen A-COO-$R^5$ (Ib)

| Beispiel Nr. Verbindung Nr. | Abweichung von A | m | $R^5$ | Ester Ib Smp. °C | Ester Ib Ausbeute % |
|---|---|---|---|---|---|
| 1 | – | 0 | n-Dodecyl | 50 | 90 |
| 2 | – | 0 | 3,7-Dimethyloct-7-en--1-yl | 53-55 | 88 |
| 3 | – | 0 | n-Tetradecyl | 62-63 | 93 |
| 4 | – | 0 | n-Hexadecyl | 66-68 | 91 |
| 5 | – | 0 | n-Octadecyl | 67-69 | 94 |
| 6 | – | 0 | n-Eikosyl | 58-60 | 90 |
| 7 | – | 0 | n-Octadec-9-enyl | 35-37 | 89 |
| 8 | – | 0 | 3,7,11,15-Tetramethylhexadecyl | Öl | 93 |
| 9 | $R^3$=H | 0 | n-Dodecyl | 57-59 | 90 |
| 10 | $R^4$= iso-Propyl | 0 | n-Dodecyl | 49-51 | 41 |
| 11 | – | 1 | n-Dodecyl | 40-43 | 85 |
| 12 | – | 1 | n-Octadecyl | 40-42 | 88 |
| 13 | – | 0 | 3,7-Dimethyloctyl | 48-49 | 81 |
| 14 | – | 0 | 3,7,11,15-Tetramethylhexadecyl | Öl | 80 |

*Beispiele 15 bis 23*

Herstellung von Estern A-$(CH_2)_m$-O-CO-$R^5$ (Ie)

In den Beispielen 15 bis 21 wurden je 30 g (0,12 Mol) des Chromanderivates A-$(CH_2)_m$-OH in 1 l Toluol und in Gegenwart von 3 g p-Toluolsulfonsäure mit der äquimolaren Menge einer Carbonsäure $R^5$-COOH so lange unter Rückflusskühlung erhitzt, bis kein Wasser mehr abgeschieden wurde. Die Aufarbeitung erfolgte wie üblich. Die Reinigung der Rohester Ie wurde durch Umkristallisation aus Methanol/Wasser vorgenommen.

In den Beispielen 22 und 23 wurden anstelle der Säuren $R^5$-COOH die entsprechenden Säurechloride eingesetzt, und die Veresterung wurde in Gegenwart der äquimolaren Menge Pyridin vorgenommen. Die Ergebnisse sind in Tabelle 2 zusammengestellt.

Tabelle 2

Verbindungen A-$(CH_2)_m$-O-CO-$R^5$ (Ie)

| Beispiel Nr. Verbindung Nr. | m | $R^5$-COOH | Ester Ie Smp. °C | Ausbeute % |
|---|---|---|---|---|
| 15 | 2 | Stearinsäure | 52 | 94 |
| 16 | 2 | Palmitinsäure | 52-54 | 93 |
| 17 | 2 | Ölsäure | Öl | 95 |
| 18 | 2 | Laurinsäure | 53-54 | 92 |
| 19 | 2 | Linolsäure | Öl | 88 |
| 20 | 2 | Linolensäure | Öl | 88 |
| 21 | 2 | Kokosfettsäure | Öl | 92 |
| 22 | 1 | Stearinsäurechlorid | 44 | 81 |
| 23 | 1 | Laurinsäurechlorid | 42 | 83 |

*Beispiele 24 und 25*

Herstellung von Carbonaten A-$(CH_2)_m$-O-CO-O-$R^5$ (If)

Analog den Beispielen 22 und 23 wurde das Chromanderivat A-$(CH_2)_m$-OH mit einem Chlorameisensäureester $R^5$-O-CO-Cl umgesetzt. Die Ergebnisse sind der Tabelle 3 zu entnehmen.

Tabelle 3

| Beispiel Nr. Verbindung Nr. | m | $R^5$ | Carbonat If Smp. °C | Ausbeute |
|---|---|---|---|---|
| 24 | 2 | n-Hexadecyl | 60 | 91 |
| 25 | 1 | n-Hexadecyl | 48-50 | 73 |

*Beispiel 26*

Herstellung eines Amids A-$CH_2$-NH-CO-$R^5$ (Ic)

4,7 g (0,02 Mol) des Aminomethylchromanderivates A-$CH_2$-$NH_2$ wurden in 150 ml Toluol in Gegenwart von 3 g Piperidin mit 5,5 g (0,02 Mol) Palmitinsäurechlorid umgesetzt. Die übliche Aufarbeitung lieferte die Verbindung A-$CH_2$-NH-CO-palmityl in 88%iger Ausbeute.

*Beispiel 27*

Herstellung eines Urethans A-$CH_2$-NH-CO-O-$R^5$ (Ij)

Analog Beispiel 26 wurde das Aminomethylchromanderivat mit 6,13 g (0,02 Mol) n-Hexadecylchlorameisensäureester umgesetzt. Die übliche Aufarbeitung lieferte das Urethan A-$CH_2$-NH-CO-O-n-hexadecyl als farbloses Öl in 90%iger Ausbeute.

*Beispiel 28*

Herstellung eines Amids A-$CH_2$-NH-CO-$R^5$ (Ic) über ein OH-geschütztes Chromanderivat

10 g (31 mMol) 6-Benzyl-A-$CH_2$-$NH_2$, welches durch Umsetzung von A-$CH_2$-$NH_2$ mit Benzylchlorid in Gegenwart von Kaliumcarbonat und Dimethylformamid hergestellt wurde (Smp. des Hydrochlorids 155°C), wurde analog Beispiel 26 mit Palmitinsäurechlorid umgesetzt und wie üblich auf das 6-Benzyl-A-$CH_2$-NH-CO-n-pentadecyl aufgearbeitet. Die Ausbeute an dieser Verbindung betrug 98%; Smp. 83 bis 84°C. Anschliessend wurde die Benzylgruppe durch Hydrierung bei 55°C und 10 bar Wasserstoffdruck in Gegenwart eines Pd/Kohle-Katalysators abgespalten. Die Aufarbeitung lieferte das freie A-$CH_2$-NH-CO-n-pentadecyl in 87%iger Ausbeute als gelbliches Öl.

*Beispiel 29*

Herstellung der Verbindung (1) nach der Methode ($b_2$)

In eine Mischung aus 9 g (0,06 Mol) Trimethylhydrochinon, 8 g (0,06 Mol) 4-Chlor-2-methylcrotonsäure, 3 g wasserfreiem Zinkchlorid und 80 ml Heptan wurden im Laufe einer Stunde bei 98°C 3 l Chlorwasserstoff eingeleitet. Nach weiterem zweistündigem Erhitzen wurde die Heptanphase dekantiert und der verbleibende ölige Rückstand mit 50 ml Methanol versetzt und 24 Stunden lang bei 20°C gerührt. Hierbei bildete sich ein Niederschlag, der abgetrennt wurde. Die verbleibende methanolische Mutterlauge wurde mit Wasser versetzt, worauf sich ein weiterer Niederschlag bildete. Beide Niederschläge wurden vereinigt und aus Wasser umkristallisiert. Die Ausbeute an 4-(2,5-Dihydroxy-3,4,6-trimethylphenyl)-2-methylcrotonsäure betrug 62%, Smp. 208°C (Zers.).

Diese Verbindung wurde nach der Vorschrift der Beispiele 1 bis 12 mit n-Dodecanol verestert. Die Ausbeute an Ester, bezogen auf die Säure, betrug 79%, Smp. 40 bis 43°C. Der Ester wurde mit 0,1

g Kupfer(I)-bis-pyridin-chlorid in 250 ml Methanol gelöst, worauf Luft durch diese Lösung geleitet wurde. Hierbei ging das Hydrochinon in das entsprechende Chinon über (gelbes Öl). Ausbeute quantitativ.

12 g des Chinonderivates wurden in 20 ml wasserfreiem Pyridin 1,5 Stunden lang unter Rückflusskühlung erhitzt, wobei sich das Chromenderivat

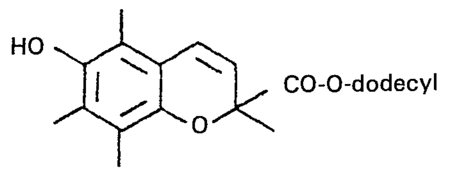

bildete; Ausbeute, bezogen auf das Chinon, 73%; Smp. 48 bis 51 °C.

Die Hydrierung zum Chromanderivat (1) wurde bei 50°C und 10 bar Wasserstoffdruck an einem Pd/Kohle-Katalysator vorgenommen. Die Ausbeute, bezogen auf das Chromen, betrug 96%.

*Versuche zur stabilisierenden Wirkung der Chromanderivate I*

1. Farbe von Polypropylen

Hier wurde die Farbqualität als Yellowness-Index YI nach dem Yellowness-Test (ASTMD 1925) gemessen.

Als Testmaterial diente additivfreies dechloriertes Polypropylen, in welches der Stabilisator auf jeweils gleiche Weise eingearbeitet wurde, und das zu Granulat von 15 mm Schichtdicke und zu Platten von 1 mm Schichtdicke geformt wurde. Die angegebenen YI-Werte sind jeweils das Mittel aus zwei Messungen. Je höher diese Werte, um so geringer ist die Farbqualität. Die Ergebnisse sind der Tabelle 4 zu entnehmen. Den Werten entsprechen etwa folgende wahrnehmbare Verfärbungen des Testmaterials:

| | |
|---|---|
| 2 | nicht erkennbare Verfärbung |
| 3- 5 | sehr schwache Verfärbung |
| 5-10 | schwache, aber bereits deutlich erkennbare Verfärbung |
| 10-20 | merkliche Verfärbung |
| 20 | starke Verfärbung |

Tabelle 4

Yellowness-Index YI von Polypropylen-Platten und -Granulat

| Versuch Nr. | Stabilisator* | gemäss Beispiel | Menge Gew.-% | YI-Index Platten | Granulat |
|---|---|---|---|---|---|
| *zum Vergleich* | | | | | |
| 1 | ohne Stabilisator | – | 0,1 | 0 | 1 |
| 2 | Q** | – | 0,1 | 1 | 8 |
| 3 | α-Tocopherol | – | 0,1 | 9 | 28 |
| 4 | $-CO-O-CH_3$ | – | 0,1 | 12 | 36 |
| 5 | -CO-O-n-heptyl | – | 0,1 | 7 | 25 |
| 6 | $-CH_2-CO-O-CH_3$ | – | 0,1 | 9 | 34 |
| 7 | Q | – | 0,1 | | |
| | + Calciumstearat | – | 0,2 | 2 | 4 |
| 8 | α-Tocopherol | – | 0,1 | | |
| | Calciumstearat | – | 0,2 | | |
| | DSDP*** | – | 0,2 | 4 | 15 |
| 9 | $-CH_2-CO-O-CH_3$ | – | 0,1 | | |
| | CA-Stearat | – | 0,2 | | |
| | DSDP | – | 0,2 | 5 | 17 |
| *erfindungsgemäss* | | | | | |
| 10 | CO-O-3,7-dimethyloctyl | 2 | 0,1 | 6 | 23 |
| 11 | -CO-O-n-dodecyl | 1 | 0,1 | 5 | 22 |
| 12 | -CO-O-3,7,11,15--tetramethylhexadecyl | 8 | 0,1 | 4 | 20 |
| 13 | $-CH_2$-CO-O-n-dodecyl | 11 | 0,1 | 9 | 30 |
| 14 | $-CH_2$-CO-O-n-octadecyl | 12 | 0,1 | 20 | 30 |
| 15 | -CO-O-oleyl | 7 | 0,1 | 7 | 23 |
| 16 | -CO-O-n-eikosyl | 6 | 0,1 | 4 | 17 |
| 17 | $-CH_2-CH_2$-O-CO-O--hexadecyl | 24 | 0,1 | 5 | 23 |
| 18 | $-CH_2$-NH-CO-n--pentadecyl | 26 | 0,1 | 4 | 22 |
| 19 | $-CH_2$-O-CO-n--heptadecyl | 22 | 0,1 | 5 | 23 |
| 20 | $-CH_2-CH_2$-O-CO-n--pentadecyl | 16 | 0,1 | 6 | 22 |

Tabelle 4 (Fortsetzung)

| Versuch Nr. | Stabilisator* | gemäss Beispiel | Menge Gew.-% | YI-Index Platten | YI-Index Granulat |
|---|---|---|---|---|---|
| 21 | $-CH_2-CH_2-O-CO-n-$ -heptadecyl | 15 | 0,1 | 5 | 22 |
| 22 | -CO-O-n-dodecyl | 1 | 0,1 | | |
| | Ca-Stearat | | 0,2 | | |
| | DSDP | | 0,2 | 2 | 8 |
| 23 | -CO-O-3,7,11,15- -tetramethylhexadecyl | 8 | 0,1 | | |
| | Ca-Stearat | | 0,2 | | |
| | DSDP | | 0,2 | 2 | 9 |
| 24 | $-CH_2-CO-O-n-$dodecyl | 11 | 0,1 | | |
| | Ca-Stearat | | 0,2 | | |
| | DSDP | | 0,2 | 6 | 18 |
| 25 | $-CH_2-CH_2-O-CO-n-$ -heptadecyl | 15 | 0,1 | | |
| | Ca-Stearat | | 0,2 | | |
| | DSDP | | 0,2 | 3 | 10 |

*) gekennzeichnet durch den Rest $-(CH_2)_m-X_n-CO-Y_r-R^5$ in der Verbindung $A-(CH_2)_m-X_n-CO-Y_r-R^5$, soweit nicht anders angegeben.
**) Neopentylglykol-[3-(3,5-di-tert.-butyl-4-hydroxyphenyl)-propionsäure]-tetraester, handelsüblich.
***) Distearylthiopropionat.

2. Verarbeitungsstabilität von Polypropylen

Die Polypropylenproben (gleiches Material wie beim Farbtest) wurden sechsmal extrudiert und granuliert. Aus den Schmelzindizes MFI (zur Bestimmungsmethode s. DIN 53 735) nach der ersten und der sechsten Extrusion wurde der Quotient $MFI_6/MFI_1$ gebildet. Je grösser dieser Quotient ist, um so geringer ist die Verarbeitungsstabilität. Die Farbmessungen entsprechen dem vom Farbtest. Die Ergebnisse sind der Tabelle 5 zu entnehmen.

Tabelle 5

Schmelzindex-Quotient $MFI_6/MFI_1$ und Yellowness-Index von Polypropylen

| Versuch Nr. | Stabilisator* | gemäss Beispiel | Menge Gew.-% | $MFI_6/MFI_1$ | YI-Index (Granulat) $YI_1$ | $YI_6$ | Δ |
|---|---|---|---|---|---|---|---|
| *zum Vergleich* | | | | | | | |
| 26 | ohne Stabilisator | — | — | 7,3 | 1 | 5 | 4 |
| 27 | Q** | — | 0,1 | 2,5 | 7 | 30 | 13 |
| 28 | α-Tocopherol | — | 0,1 | 2,1 | 28 | 38 | 10 |
| 29 | $-CO-O-CH_2$ | — | 0,1 | 1,9 | 36 | 46 | 10 |
| 30 | -CO-O-n-heptyl | — | 0,1 | 1,9 | 25 | 32 | 7 |
| 31 | $-CH_2-CO-O-CH_3$ | — | 0,1 | 2,2 | 34 | 44 | 10 |
| 32 | Q | — | 0,1 | | | | |
| | Ca-Stearat | — | 0,2 | 2,9 | | | |
| 33 | α-Tocopherol | — | 0,1 | | | | |
| | DSDP*** | — | 0,2 | | | | |
| | Ca-Stearat | — | 0,2 | 1,5 | 15 | 20 | 5 |
| *erfindungsgemäss* | | | | | | | |
| 34 | -CO-O-3,7-dimethyl-octyl | 2 | 0,1 | 1,9 | 23 | 32 | 9 |
| 35 | -CO-O-n-dodecyl | 1 | 0,1 | 1,6 | 22 | 31 | 9 |
| 36 | -CO-O-n-tetradecyl | 3 | 0,1 | 1,6 | 18 | 33 | 15 |
| 37 | -CO-O-n-hexadecyl | 4 | 0,1 | 1,6 | 22 | 32 | 10 |
| 38 | -CO-O-n-octadecyl | 5 | 0,1 | 1,6 | 18 | 32 | 14 |
| 39 | -CO-O-n-eikosyl | 6 | 0,1 | 1,6 | 18 | 31 | 13 |
| 40 | -CO-O-oleyl | 7 | 0,1 | 1,6 | 23 | 40 | 17 |
| 41 | -CO-O-3,7,11,15-tetra-methylhexadecyl | 8 | 0,1 | 1,6 | 20 | 28 | 8 |
| 42 | $-CH_2-CO-n-$dodecyl | 11 | 0,1 | 1,9 | 30 | 38 | 8 |
| 43 | $-CH_2-CO-n-$octadecyl | 12 | 0,1 | 1,7 | | | |

Tabelle 5 (Fortsetzung)

| Versuch Nr. | Stabilisator* | gemäss Beispiel | Menge Gew.-% | $MFI_6/MFI_1$ | YI-Index (Granulat) $YI_1$ | $YI_6$ | Δ |
|---|---|---|---|---|---|---|---|
| 44 | $-CH_2-CH_2-O-CO-O-n-$ -hexadecyl | 24 | 0,1 | 1,6 | | | |
| 45 | $-CH_2-O-CO-n-$ -heptadecyl | 22 | 0,1 | 1,6 | 24 | 37 | 13 |
| 46 | $-CH_2-CH_2-O-CO-n-$ -heptadecyl | 15 | 0,1 | 1,6 | 22 | 36 | 14 |
| 47 | -CO-O-n-dodecyl | 1 | 0,1 | | | | |
| | Ca-Stearat | | 0,2 | | | | |
| | DSDP | | 0,2 | 1,7 | 8 | 26 | 18 |
| 48 | -CO-O-3,7,11,15-tetra-methylhexadecyl | 8 | 0,1 | | | | |
| | Ca-Stearat | | 0,2 | | | | |
| | DSDP | | 0,2 | 1,7 | 9 | 26 | 17 |
| 49 | $-CH_2-CO-O-n-$dodecyl | 11 | 0,1 | | | | |
| | Ca-Stearat | | 0,2 | | | | |
| | DSDP | | 0,2 | 1,7 | 19 | 37 | 16 |
| 50 | $-CH_2-CH_2-O-CO-n-$ -heptadecyl | 15 | 0,1 | | | | |
| | Ca-Stearat | | 0,2 | | | | |
| | DSDP | | 0,2 | 1,7 | 10 | 24 | 14 |

* ** *** Vergl. Fussnoten zu Tabelle 4

3. Langzeitstabilität von Polypropylen

3.1 Ofenalterung

Polypropylenplatten der Beschaffenheit wie beim Farbtest wurden nach DIN 53 383, Blatt 1, der sogenannten Ofenalterung unterworfen, indem die Platten im Wärmeschrank unter Frischluftzufuhr so lange auf 140°C erhitzt wurden, bis sie erkennbar versprödeten. Die visuelle Prüfung wurde alle 24 Stunden vorgenommen, d.h. die Alterung wurde hier in Tagen gemessen. Je geringer die Werte, desto geringer die Langzeitstabilität. Die Werte sind Mittelwerte aus 10 Messungen und verstehen sich jeweils mit einer Abweichung mit bis zu etwa 5%. Die Ergebnisse sind in Tabelle 6 zusammengestellt.

3.2 Sauerstoffaufnahme bei 180°C

Polypropylenproben wurden in geschmolzenem Zustand unter reiner Sauerstoffatmosphäre auf 180°C bis zum Beginn eines merklichen Sauerstoffverbrauchs erhitzt. Diese sogenannte Induktionszeit wurde in Minuten gemessen. Tabelle 7 gibt die Ergebnisse wieder.

Tabelle 6

Ofenalterung von Polypropylen bei 140°C

| Versuch Nr. | Stabilisator* | gemäss Beispiel Nr. | Menge Gew.-% | Alterungszeit (Tage) |
|---|---|---|---|---|
| *zum Vergleich* | | | | |
| 51 | ohne Stabilisator | – | – | 1 |
| 52 | Q** | – | 0,1 | 40 |
| 53 | α-Tocopherol | – | 0,1 | 3 |
| 54 | $-CO-O-CH_3$ | – | 0,1 | 1 |
| 55 | -CO-O-n-heptyl | – | 0,1 | 1 |
| 56 | $-CH_2-CO-O-$methyl | – | 0,1 | 1 |
| 57 | Q | – | 0,1 | |
| | Ca-Stearat | – | 0,2 | 31 |
| 58 | α-Tocopherol | - | 0,1 | |
| | Ca-Stearat | | | |
| | DSDP*** | – | 0,2 | 14 |
| *erfindungsgemäss* | | | | |
| 59 | -CO-O-3,7-dimethyloctyl | 2 | 0,1 | 1 |
| 60 | -CO-O-n-dodecyl | 1 | 0,1 | 3 |

Tabelle 6 (Fortsetzung)

| Versuch Nr. | Stabilisator* | gemäss Beispiel Nr. | Menge Gew.-% | Alterungszeit (Tage) |
|---|---|---|---|---|
| 61 | -CO-O-n-tetradecyl | 3 | 0,1 | 3 |
| 62 | -CO-O-n-hexadecyl | 4 | 0,1 | 3 |
| 63 | -CO-O-n-octadecyl | 5 | 0,1 | 3 |
| 64 | -CO-O-n-eikosyl | 6 | 0,1 | 3 |
| 65 | -CO-O-n-oleyl | 7 | 0,1 | 3 |
| 66 | -CO-O-3,7,11,15-tetramethyl-hexadecyl | 8 | 0,1 | 3 |
| 67 | -$CH^2$-CO-O-n-dodecyl | 11 | 0,1 | 1 |
| 68 | -$CH_2$-CO-O-n-octadecyl | 12 | 0,1 | 1 |
| 69 | -$CH_2$-O-CO-n-heptadecyl | 22 | 0,1 | 3 |
| 70 | -$CH_2$-$CH_2$-O-CO-heptadecyl | 15 | 0,1 | 3 |
| 71 | -$CH_2$-$CH_2$-O-CO-O-n-hexadecyl | 24 | 0,1 | 3 |
| 72 | -CO-NH-n-dodecyl | — | 0,1 | 3 |
| 73 | -CO-S-n-dodecyl | — | 0,1 | 3 |
| 74 | -$CH_2$-NH-CO-n-pentadecyl | — | 0,1 | 3 |
| 75 | -CO-O-3,7,11,15-tetramethyl-hexadecyl | 8 | 0,1 | |
| | Ca-Stearat | — | 0,2 | |
| | DSDP | — | 0,2 | 18 |
| 76 | -$CH_2$-CO-O-n-dodecyl | 11 | 0,1 | |
| | Ca-Stearat | — | 0,2 | |
| | DSDP | — | 0,2 | 17 |
| 77 | -$CH_2$-$CH_2$-O-CO-n-heptadecyl | 15 | 0,1 | |
| | Ca-Stearat | — | 0,2 | |
| | DSDP | — | 0,2 | 19 |

* ** *** Vergl. Fussnoten zu Tabelle 4

Tabelle 7

Sauerstoffaufnahme von Polypropylenschmelzen bei 180°C

| Versuch Nr. | Stabilisator* | gemäss Beispiel | Menge Gew.-% | Induktions-zeit (min) |
|---|---|---|---|---|
| *zum Vergleich* | | | | |
| 78 | ohne Stabilisator | — | — | 6 |
| 79 | Q** | — | 0,1 | 77 |
| 80 | -CO-O-$CH_3$ | — | 0,1 | 98 |
| 81 | -CO-O-n-heptyl | — | 0,1 | 55 |
| *erfindungsgemäss* | | | | |
| 82 | -CO-O-3,7-dimethyloctyl | 2 | 0,1 | 63 |
| 83 | -CO-O-n-dodecyl | 1 | 0,1 | 79 |
| 84 | -CO-O-n-eikosyl | 6 | 0,1 | 76 |
| 85 | -$CH_2$-$CH_2$-O-CO-n--heptadecyl | 15 | 0,1 | 73 |

* ** *** Vergl. Fussnoten zu Tabelle 4

4. Stabilität von β-Carotin

4.1 Photostabilität

Je 10 mg eines Trockenpulvers, welches aus 88,5 Gew.-% Polyvinylpyrrolidon, 10 Gew.-% β-Carotin und 1,5 Gew.-% eines Stabilisators bestand, wurden in 10 ml luftgesättigtem Wasser gelöst. Der intensiven Gelbfärbung der klaren Lösung entsprach ein Licht-Absorptionsmaximum von 430 nm. Bei dieser Wellenlänge und 1 cm Schichtdicke der Lösung betrug die Extinktion 0,8, die gleich 100% gesetzt wurde.

Zur Ermittlung der Stabilität des β-Carotins gegen den Einfluss von Licht wurde die Lösung mit monochromatischem UV-Licht einer Quecksilberhochdrucklampe bestrahlt (365 nm; 1 $mW/cm^2$). Ein Mass für die Zerstörung des β-Carotins durch diese Bestrahlung ist die Entfärbung der Lösung, also die Abnahme der Extinktion. Der Tabelle 8 ist jeweils diejenige Zeit in Minuten zu entnehmen, bei der die Extinktion auf den 1/e-ten Teil, also auf rund 0,4% abfiel. Näheres zu dieser Bestimmungsmethode vgl. J. Photochem., Band 7 (1977), S. 355.

4.2 Lagerstabilität

Das β-Carotin-Trockenpulver obiger Zusammensetzung wurde 7 Tage lang im Dunkeln bei 25°C gelagert. Anschliessend wurde die Extinktion in Prozent des ursprünglichen Wertes $E_o$ (= 100%) bestimmt. Für die Messungen wurden jeweils Lösungen aus 50 mg des Trockenpulvers in 100 ml Chloroform verwendet. Die Ergebnisse dieses Tests gehen ebenfalls aus der Tabelle 8 hervor.

Tabelle 8

Photostabilität und Lagerstabilität von β-Carotin (Stabilisatorkonzentration, bezogen auf Carotin, jeweils 15 Gew.-%)

| Versuch Nr. | Stabilisator* | gemäss Beispiel min | Photo-stabilität % von E | Lager stabilität nach 7 Tagen |
|---|---|---|---|---|
| *zum Vergleich* | | | | |
| 86 | ohne Stabilisator | – | 5 | 10 |
| 87 | α-Tocopherol | – | 75 | 31 |
| 88 | $-CO-O-CH_3$ | – | 15 | 32 |
| 89 | -CO-O-n-heptyl | – | 62 | 30 |
| 90 | -COOH | – | 18 | 59 |
| 91 | Ascorbylpalmitat | – | 22 | 20 |
| *erfindungsgemäss* | | | | |
| 92 | -CO-O-n-dodecyl | 1 | 105 | 35 |
| 93 | -CO-O-n-tetradecyl | 3 | 104 | 37 |
| 94 | -CO-O-3,7,11,15-tetramethylhexadecyl | 8 | 87 | 34 |
| 95 | -CO-O-n-eikosyl | 6 | 86 | 35 |
| 96 | $-CH_2-O-CO-$n-heptadecyl | 22 | 106 | 45 |
| 97 | $-CH_2-CH_2-O-CO-$n-heptadecyl | 15 | 77 | 52 |
| 98 | $-CH_2-CH_2-O-CO-O-$n-hexadecyl | 24 | 97 | 47 |
| 99 | -CO-NH-n-dodecyl | – | 80 | 40 |

* Vergl. Fussnote zu Tabelle 4

**Patentansprüche**

1. Chromanderivate der allgemeinen Formel I

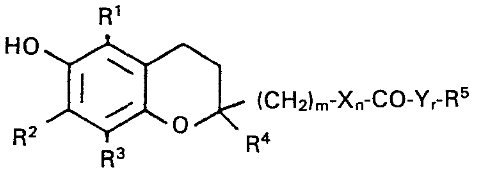

in der die Substituenten und Indizes folgende Bedeutung haben:

$R^1$-$R^4$ H, $C_1$-$C_4$-Alkyl
$R^5$ $C_{10}$-$C_{30}$-Alkyl oder -Alkenyl
X, Y O, NH, S
m 0, 1, 2 oder 3
n, r 0 oder 1

2. Chromanderivate gemäss Anspruch 1, wobei $R^1$-$R^4$-Methylgruppen und X und/oder Y Sauerstoff bedeuten.

3. Verfahren zur Herstellung von Chromanderivaten Ia (n = r = 0), dadurch gekennzeichnet, dass man ein Säurehalogenid IIa

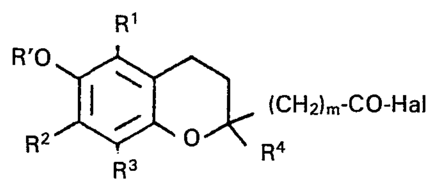

IIa

wobei R' eine Schutzgruppe ist, in an sich bekannter Weise in Gegenwart von $CdCl_2$ mit einer Grignard-Verbindung $R^5$-Mg-Hal umsetzt und das Addukt unter Abspaltung der Schutzgruppe R' hydrolysiert.

4. Verfahren zur Herstellung von Chromanderivaten Ib (n = 0, Y = O), Ic (n = 0, Y = NH) und Id

(n = 0, Y = S), dadurch gekennzeichnet, dass man eine Säure IIb

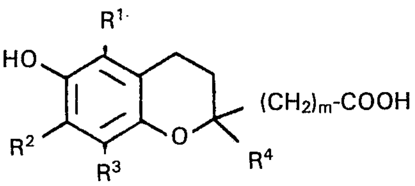

IIb

oder ein funktionelles Derivat dieser Säure in an sich bekannter Weise mit einem Alkohol $R^5$-OH (IIIb), einem Amin $R^5$-$NH_2$ (IIIc) bzw. einem Thiol $R^5$-SH (IIId) oder einem funktionellen Derivat von IIIb, IIIc bzw. IIId umsetzt.

5. Verfahren zur Herstellung von Chromanderivaten Ie (X = O, r = O), If (X = O, Y = O), Ig (X = O, Y = NH) und Ih (X = O, Y = S), dadurch gekennzeichnet, dass ein Chromanderivat IIe

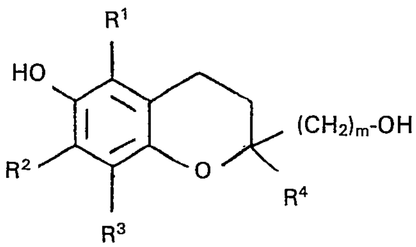

IIe

in an sich bekannter Weise mit einer Säure $R^5$-CO-OH (IIIe), einem Halogenameisensäureester $R^5$-O--CO-Hal (IIIf), einem Carbaminsäurehalogenid $R^5$--NH-CO-Hal (IIIg) bzw. einem Halogenameisensäurethioester $R^5$-S-CO-Hal (IIIh) oder mit funktionell äquivalenten Derivaten dieser Verbindungen umsetzt.

6. Verfahren zur Herstellung von Chromanderivaten Ii (X = NH, r = O), Ij (X = NH, Y = O), Ik (X = NH, Y = NH) und Il (X = NH, Y = S), dadurch gekennzeichnet, dass man ein Chromanderivat IIi

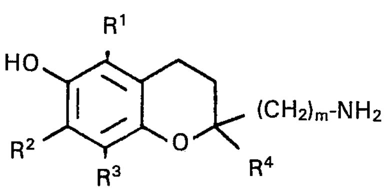

IIi

in an sich bekannter Weise mit einer Säure $R^5$-CO-OH (IIIe), einem Halogenameisensäureester $R^5$-O--CO-Hal (IIIf), einem Carbaminsäurehalogenid $R^5$--NH-CO-Hal (IIIg) bzw. einem Halogenameisensäurethioester $R^5$-S-CO-Hal (IIIh) oder mit funktionell äquivalenten Derivaten dieser Verbindungen umsetzt.

7. Verfahren zur Herstellung von Chromanderivaten Im (X = S, r = O), In (X = S, Y = O), Io (X = S, Y = NH) und Ip (X = S'', Y = S), dadurch gekennzeichnet, dass man ein Chromanderivat IIm

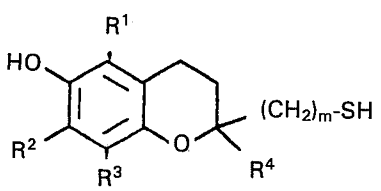

IIm

in an sich bekannter Weise mit einer Säure $R^5$-CO-OH (IIIe), einem Halogenameisensäureester $R^5$-O--CO-Hal (IIIf), einem Carbaminsäurehalogenid $R^5$--NH-CO-Hal (IIIg) bzw. einem Halogenameisensäurethioester $R^5$-S-CO-Hal (IIIh) oder mit funktionell äquivalenten Derivaten dieser Verbindungen umsetzt.

8. Verfahren zur Herstellung der Chromanderivate Ig, Ik und Io, dadurch gekennzeichnet, dass man ein Chromanderivat IIe, IIi bzw. IIm mit einem Isocyanat IV

$$R^5\text{-N}=\text{C}=\text{O} \qquad \text{IV}$$

umsetzt.

9. Verfahren nach den Ansprüchen 4 bis 8, dadurch gekennzeichnet, dass man die 6-OH-Gruppe in den Ausgangsverbindungen, den Chromanderivaten II, vor der Umsetzung in an sich bekannter Weise durch eine Schutzgruppe schützt und diese im letzten Syntheseschritt wieder abspaltet.

10. Verwendung der Chromanderivate I zum Stabilisieren von organischen Materialien gegen schädigende Einflüsse von Licht, Wärme und Oxidationsmitteln.

11. Verwendung der Chromanderivate I gemäss Anspruch 10 zum Stabilisieren von Kunststoffen in Konzentrationen von 0,01 bis 1,0 Gew.-%, bezogen auf die Menge des Kunststoffes.

12. Verwendung der Chromanderivate I gemäss Anspruch 10 zum Stabilisieren von hochempfindlichen organischen Materialien in Konzentrationen von 0,01 bis 20 Gew.-%, bezogen auf die Menge dieses Materials.

13. Organische Materialien, gekennzeichnet durch einen Gehalt von 0,01 bis 20 Gew.-% eines Chromanderivates I als Stabilisierungsmittel.

14. Organische Materialien gemäss Anspruch 13, gekennzeichnet durch einen Gehalt von 50 bis 500 Gew.-%, bezogen auf die Menge des Chromanderivates I, eines synergistisch wirkenden Stabilisierungshilfsmittels.

**Claims**

1. A chroman derivative of the general formula I

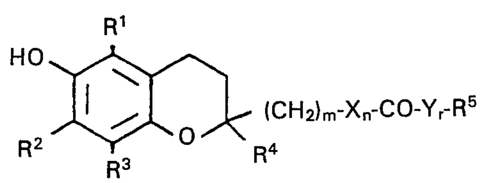

where $R^1$, $R^2$, $R^3$ and $R^4$ are each H or $C_1$-$C_4$-alkyl, $R^5$ is $C_{10}$-$C_{30}$-alkyl or $C_{10}$-$C_{30}$-alkenyl, X and Y are each O, NH or S, m is 0, 1, 2 or 3 and n and r are each 0 or 1.

2. A chroman derivative as claimed in claim 1, where $R^1$, $R^2$, $R^3$ and $R^4$ are each methyl and X and/or Y is oxygen.

3. A process for the preparation of a chroman derivative Ia (n = r = 0), wherein an acid halide IIa

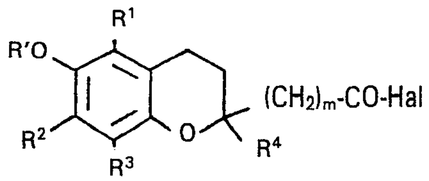

IIa

where R′ is a protective group, is reacted in a conventional manner with a Grignard compound $R^5$-Mg-Hal in the presence of $CdCl_2$, and the adduct is hydrolyzed, with elimination of the protective group R′.

4. A process for the preparation of a chroman derivative Ib (n = 0, Y = O), Ic (n = 0, Y = NH) or Id (n = 0, Y = S), wherein an acid IIb

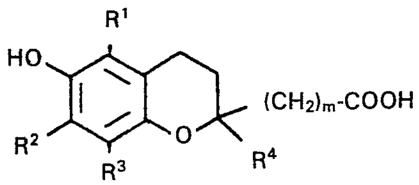

IIb

or a functional derivative of this acid is reacted, in a conventional manner, with an alcohol $R^5$-OH (IIIb), an amine $R^5$-$NH_2$ (IIIc) or a thiol $R^5$-SH (IIId) respectively, or with a functional derivative of IIIb, IIIc or IIId respectively.

5. A process for the preparation of a chroman derivative Ie (X = O, r = 0), If (X = O, Y = O), Ig (X = O, Y = NH) or Ih (X = O, Y = S), wherein a chroman derivative IIe

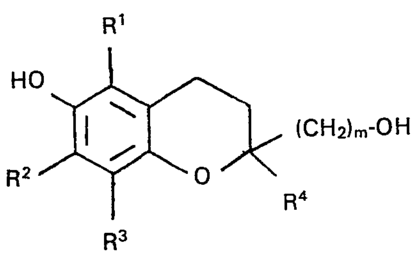

IIe

is reacted, in a conventional manner, with an acid $R^5$-COOH (IIIe), a haloformic acid ester $R^5$-O-CO-Hal (IIIf), a carbamyl halide $R^5$-NH-CO-Hal (IIIg) or a haloformic acid thioester $R^5$-S-CO-Hal (IIIh) respectively, or with a functionally equivalent derivative of these compounds.

6. A process for the preparation of a chroman derivative Ii (X = NH, r = 0), Ij (X = NH, Y = O), Ik (X = NH, Y = NH) or Il (X = NH, Y = S), wherein a chroman derivative IIi

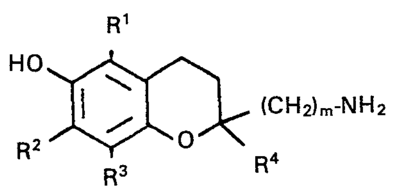

IIi

is reacted, in a conventional manner, with an acid $R^5$-COOH (IIIe), a haloformic acid ester $R^5$-O-CO-Hal (IIIf), a carbamyl halide $R^5$-NH-CO-Hal (IIIg) or a haloformic acid thioester $R^5$-S-CO-Hal (IIIh) respectively, or with a functionally equivalent derivative of these compounds.

7. A process for the preparation of a chroman derivative Im (X = S, r = 0), In (X = S, Y = O), Io (X = S, Y = NH) or Ip (X = S″, Y = S), wherein a chroman derivative IIm

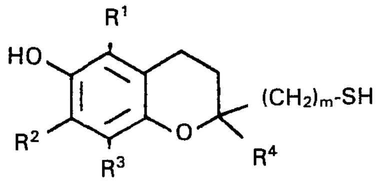

IIm

is reacted, in a conventional manner, with an acid $R^5$-COOH (IIIe), a haloformic acid ester $R^5$-O-CO-Hal (IIIf), a carbamyl halide $R^5$-NH-CO-Hal (IIIg) or a haloformic acid thioester $R^5$-S-CO-Hal (IIIh) respectively, or with a functionally equivalent derivative of these compounds.

8. A process for the preparation of a chroman derivative Ig, Ik or Io, wherein a chroman derivative IIe, IIi or IIm, respectively, is reacted with an isocyanate IV

$$R^5\text{-}N=C=O \qquad IV$$

9. A process as claimed in claims 4 to 8, wherein the 6-OH group in the starting compound, namely the chroman derivative II, is protected in a conventional manner by a protective group prior to the reaction, and is eliminated again in the last step of the synthesis.

10. The use of a chroman derivative I for stabilizing organic materials against the damaging effects of light, heat and oxidizing agents.

11. The use of a chroman derivative I as claimed in claim 10 for stabilizing plastics materials, in concentrations of from 0.01 to 1.0% by weight, based on the amount of plastics material.

12. The use of a chroman derivative I as claimed in claim 10 for stabilizing highly sensitive organic materials, in concentrations of from 0.1 to 20% by weight, based on the amount of the material.

13. Organic materials containing from 0.1 to 20% by weight of a chroman derivative I as stabilizer.

14. Organic materials as claimed in claim 13, containing 50 to 500% by weight, based on the amount of chroman derivative I, of a synergistic stabilizing assistant.

**Revendications**

1. Dérivés chromanniques de formule générale I

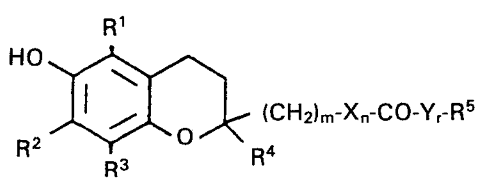

dans laquelle les substituants et indices ont les significations suivantes:

$R^1$ à $R^4$ H, alcoyle en $C_1$ à $C_4$
$R^5$ Alcoyle ou alcényle en $C_{10}$ à $C_{30}$
X, Y O, NH, S
m 0, 1, 2 ou 3
n, r 0 ou 1.

2. Dérivés chromanniques selon la revendication 1, caractérisé en ce que $R^1$ à $R^4$ représentent des groupes méthyle et X et/ou Y représentent des atomes d'oxygène.

3. Procédé pour la préparation de dérivés chromanniques Ia (n = r = 0), caractérisé en ce qu'on fait réagir un halogénure d'acide IIa

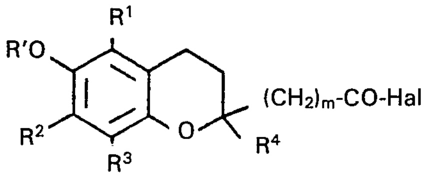
IIa

dans laquelle R' est un groupe protecteur, de façon connue en soi en présence de $CdCl_3$, avec un composé de Grignard $R^5$-Mg-Hal et on hydrolyse le produit d'addition avec élimination du groupe protecteur R'.

4. Procédé pour la préparation de dérivés chromanniques Ib (n = 0, Y = O), Ic (n = 0, Y = NH) et Id (n = 0, Y = S), caractérisé en ce qu'on fait réagir un acide IIb

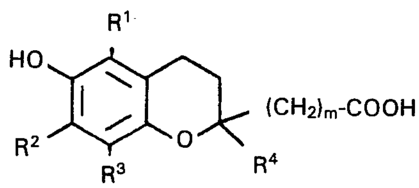
IIb

ou un dérivé fonctionnel de cet acide, de façon connue en soi, avec un alcool $R^5$-OH (IIIb), une amine $R^5$-$NH_2$ (IIIc) ou, respectivement, un thiol $R^5$-SH (IIId) ou avec un dérivé fonctionnel de IIIb, IIIc ou IIId.

5. Procédé pour la préparation de dérivés chromanniques Ie (X = O, r = 0), If (X = O, Y = O), Ig (X = O, Y = NH) et Ih (X = O, Y = S), caractérisé en ce qu'on fait réagir un dérivé chromannique IIe

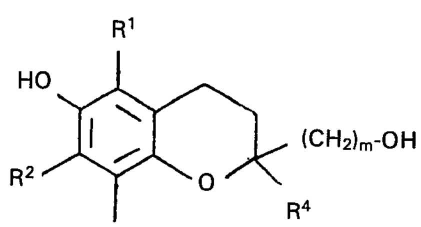
IIe

de façon connue en soi, respectivement avec un acide $R^5$-COOH (IIIe), un ester d'acide formique halogéné $R^5$-O-CO-Hal (IIIf), un halogénure d'acide carbamique $R^5$-NH-CO-Hal (IIIg) ou un thioester d'acide formique halogéné $R^5$-S-CO-Hal (IIIh) ou avec des dérivés fonctionnellement équivalents de ces composés.

6. Procédé pour la préparation de dérivés chromanniques Ii (X = NH, r = 0), Ij (X = NH, Y = O), Ik (X = NH, Y = NH) et Il (X = NH, Y = S), caractérisé en ce qu'on fait réagir un dérivé chromannique IIi

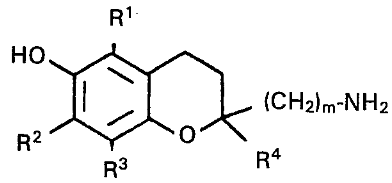
IIi

de façon connue en soi, respectivement avec un acide $R^5$-COOH (IIIe), un ester d'acide formique halogéné $R^5$-O-CO-Hal (IIIf), un halogénure d'acide carbamique $R^5$-NH-CO-Hal (IIIg) ou un thioester d'acide formique halogéné $R^5$-S-CO-Hal (IIIh) ou avec des dérivés fonctionnellement équivalents de ces composés.

7. Procédé pour la préparation de dérivés chromanniques Im (X = S, r = 0), In (X = S, Y = O), Io (X = S, Y = NH) et Ip (X = S, Y = S), caractérisé en ce qu'on fait réagir un dérivé chromannique IIm

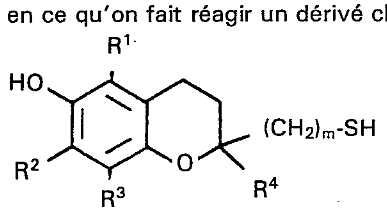
IIm

de façon connue en soi, respectivement avec un acide $R^5$-COOH (IIIe), un ester d'acide formique halogéné $R^5$-O-CO-Hal (IIIf), un halogénure d'acide carbamique $R^5$-NH-CO-Hal (IIIg) ou un thioester d'acide formique halogéné $R^5$-S-CO-Hal (IIIh) ou avec des dérivés fonctionnellement équivalents de ces composés.

8. Procédé pour la préparation des dérivés chromanniques Ig, Ik et Io, caractérisé en ce qu'on fait réagir un dérivé chromannique IIe, IIi ou IIm respectivement avec un isocyanate IV

$$R^5\text{-}N=C=O \qquad (IV)$$

9. Procédé selon l'une quelconque des revendications 4 à 8, caractérisé en ce qu'avant la réaction, on protège de façon connue en soi le groupe OH en position 6 dans les composés de départ, c'est-à-dire les dérivés chromanniques II, par un groupe protecteur et que l'on élimine ce dernier dans la dernière phase de la synthèse.

10. Utilisation des dérivés chromanniques I pour la stabilisation de matières organiques contre les effets nuisibles de la lumière, de la chaleur et d'agents d'oxydation.

11. Utilisation des dérivés chromanniques I selon la revendication 10 pour la stabilisation de matières synthétiques, dans des concentrations, rapportées à la quantité de la matière synthétique, de 0,01 à 1,0% en poids.

12. Utilisation des dérivés chromanniques I selon la revendication 10 pour la stabilisation de matières organiques ultrasensibles, dans des concentrations, rapportées à la quantité de cette matière, de 0,01 à 20% en poids.

13. Matières organiques, caractérisé par une teneur de 0,01 à 20% en poids d'un dérivé chromannique I servant de stabilisant.

14. Matières organiques selon la revendication 13, caractérisées par une teneur, rapportée à la quantité du dérivé chromannique, de 50 à 500% en poids d'un agent auxiliaire de stabilisation à effet synergique.